# EUROPEAN PATENT SPECIFICATION

(11) **EP 3 730 107 B1**
(45) Date of publication and mention of the grant of the patent: **07.06.2023**
(21) Application number: 19171422.9
(22) Date of filing: 26.04.2019
(51) Int. Cl.: A61F 11/08, A61F 11/12

(54) **AN EAR PLUG FOR HUMAN EARS, AN EAR PLUG SYSTEM, A CORDED EAR PLUG**
OHRSTÖPSEL FÜR MENSCHLICHE OHREN, OHRSTÖPSELSYSTEM, SCHNURGEBUNDENER OHRSTÖPSEL
BOUCHON D'OREILLE POUR OREILLES HUMAINES, SYSTÈME DE BOUCHON D'OREILLE, BOUCHON D'OREILLE À CORDON

(43) Date of publication of application: 28.10.2020
(73) Proprietor: Moldex/Metric AG & Co. KG, 72141 Walddorfhäslach (DE)
(72) Inventor: THIEL, Dietmar, 72141 Walddorfhäslach (DE)
(74) Representative: Clarenbach, Carl-Philipp

(56) References cited:
- EP-A1- 2 517 679
- WO-A1-2005/122981
- WO-A1-2019/043626
- US-A- 5 711 313
- US-A1- 2012 073 583
- US-B2- 8 061 472

## Description

The present invention is defined in the appended claims and concerns an ear plug for human ears, comprising a flexible nose portion for insertion into an ear, a handle portion extending longitudinal from a first end to a second end, being connected to the nose portion on the first end and comprising a fixation structure for fixating a cord to the ear plug on the second end.

Furthermore, the invention refers to an ear plug system for human beings that comprises at least one ear plug and at least one cord having at least one stiffened end which is attachable to the at least one ear plug.

Also, the present invention refers to a corded ear plug made of the above described ear plug system.

Ear plugs, ear plug systems and corded ear plugs are known in the art. For example, the published patent application EP 2 517 679 describes that common ear plugs are generally made by initially making an ear plug by any conventional technique provided with an elongated opening at one end, whereby an adhesive is then applied to the opening and one end of a cord is inserted into the elongated opening during the time that the adhesive is still liquid. The adhesive then bonds the cord within the opening. Generally, a pair of such ear plug is located at opposite ends of the cord. The document proposes to provide a pair of openings physically displaced from each other on one end of the ear plug such that a cord that is arranged within the openings is physically and/or angularly displaced along its length to lock the flexible cord material to the ear plug.

The published patent application US 2012/073583 A1 discloses an apparatus and a method of making a corded earplug, including a flexible cord material and a pair of earplugs. Each earplug has a nose portion for insertion into the ear and a rear portion extending from the nose portion and lying outside of the ear when the nose portion is inserted into the ear. Each rear portion has a pair of openings physically displaced from each other and/or angularly displaced from each other. At least one of the openings extends through the rear portion. A short portion of the flexible cord material is inserted through the at least one opening extending through the rear portion. The short portion of the flexible cord material is then inserted into the other opening to have the short portion of the flexible cord material physically displaced and/or angularly displaced along its length to lock the flexible cord material to the rear portion of the earplug.

It is the object of the present invention to provide a better ear plug for human ears that allows a strong and easy to mount or to assemble connection of a flexible cord with the ear plug.

The object of the present invention is obtained by an ear plug with the features of claim 1. It has the advantage that by a smart arrangement of openings at the second end of the ear plug a cord can be safely attached to the ear plug without additional auxiliary means in an easy and time efficient way. According to the present invention, the fixation structure on the second end comprises a first guide channel oriented at least essentially parallel to the longitudinal extension of the handle portion, the first guide channel leading to a first opening at a front surface of the second end, the fixation structure further comprises a second guide channel that extends from a second opening in an outer mantle surface of the handle portion through a third opening into the first channel and that is oriented transverse to the first guide channel such that the second guide channel is aligned with the first opening. Thus, according to the invention, the first guide channel is oriented at least substantially parallel to the longitudinal extension of the handle portion and as such extends longitudinal itself in particular from a bottom within the handle portion to the first opening on the front surface at the second end of the handle portion. The second guide channel is oriented transverse to the first guide channel, for example perpendicular to the longitudinal extension of the first guide channel or aligned to the first guide channel in an angle smaller or greater than 90°. In any case, the second guide channel is oriented such that it leads from the second opening in the outer mantle surface of the handle portion into the first guide channel such that in the longitudinal extension of the second guide channel the second guide channel is aligned with the first opening. Herewith, an end of the cord which is to be fixed on the ear plug can be pushed through the second guide channel such that it exits the handle portion at the first opening by passing through the first guide channel. This design allows in particular a cord with a stiffened end segment to be pushed through the second guide channel from the second hole out of the first guide channel pulling the cord behind and then being pushed back directly into the first guide channel through the first opening, preferably until the stiffened end segment lies beyond the third opening such that the flexible cord reaches through the second guide channel only. Now, the flexible cord is displaced in its longitudinal extension and it is impossible to pull the stiffened end segment out of the first guide channel of the handle portion by pulling on the flexible part of the cord that reaches through the second guide channel to the outside. Therefore, the cord is fixed/attached to the handle portion securely without the need for auxiliary means or a complicated mounting process of the cord.

According to a preferred embodiment of the present invention, the distance from the second opening to the first opening is shorter than the distance from the first or third opening to the front end of the first guide channel being opposite to the first opening. With this design, it is guaranteed that a stiffened end of a cord cannot simply be bend into the first guide channel when exiting the third opening and that the stiffening end may be arranged fully within the first guide channel. Preferably, the length of the first guide channel from the third opening to the end of the guide channel is at least as long as the stiffened end segment of the cord which is to be attached ear plug such that a flexible portion of the cord is displaced/displaceable at least essentially right-angled from the second guide channel into the first guide channel.

According to a further preferred embodiment of the present invention, the front surface of the second end of the handle portion extends in a plane that is oriented inclined to a perpendicular orientation to the longitudinal extension of the first guide channel and/or of the handle portion. By providing an inclined front surface which comprises the first opening, the orientation of the second guide channel may be altered such that it is oriented perpendicular to the longitudinal extension of the first guide channel. The first opening is then, due to the inclined front surface, more of oval shape. With this design, a free end of the cord, in particular the stiffened end segment of the cord may be pushed through the second guide channel for example perpendicular to the first guide channel and out of the first opening in a linear motion. Thus, the tilted or inclined front surface allows for a more perpendicular oriented second guide channel. It is particularly preferred that both, the second guide channel as well as the front surface are inclined towards a plane perpendicular to the longitudinal extension of the handle portion such that the second guide channel is aligned with the first opening. After pushing the end of the cord into the first guide channel through the first opening directly, the cord may comprise a 90° or smaller displacement that leads from the second guide channel to the first guide channel, thereby providing an advantageous mechanical attachment of the cord to the ear plug. The design also allows for an easy exchange of the ear plug.

Furthermore, a cross-section of the first channel is preferably larger than a cross-section of the second channel. While the second guide channel only needs to be as wide as the cross-section of the cord and in particular its stiffened end portion, the first guide channel needs to provide a larger cross-section, since both, a part of the cord as well as the stiffened end of the cord have to find room within the first channel when the stiffened end segment of the cord is pushed directly through the first opening into the first guide channel after the cord was pulled through the second guide channel exiting the first opening. However, the cross-section of the first channel is preferably at least as wide as the stiffened segment of the cord and a flexible cord section next to one another only, in order to provide a safe fastening of the cord to the handle portion and to aggravate removing the cord from the fixation structure. Preferably, the cross-section of the first guide channel is constant over the length or most of the length of the first guide channel. Alternatively, the first guide channel is of conical design with a cross-section that decreases in the direction from the first opening to the end of the first guide channel. Hence, the first guide channel is preferably cylindrical or conical shaped in design.

Furthermore, the second guide channel is inclined to a plane perpendicular to the longitudinal extension of the first guide channel or a handle portion. With the inclined orientation of the second guide channel, the inclination of the front surface may be smaller which can lead to advantages in light of design and cosmetics.

The inventive ear plug system with features of present claim 7 is characterized in that it comprises at least one flexible cord, preferably a large number of equal or unequal cords, each with at least one stiffened end segment, and at least one inventive ear plug, preferably a large number of equal or different sized ear plugs of the inventive type that are attachable to the at least one stiffened end of the cord. The cord can be attached to the ear plug, in particular to the fixation structure of the ear plug, in the way described above, by pushing the stiffened end through the second guide channel from the second opening through the first guide channel and out of the first opening and subsequently pushing it back and directly through the first opening into this first guide channel until the stiffened end portion is arranged beyond the third opening. This leads to the advantages that have already been discussed above.

It is particularly preferred that the first guide channel and/or the stiffened end segment are designed such that the end segment can fully be arranged in the first guide channel between the third opening and said front end of the first guide channel. According to this embodiment, the stiffened end segment of the cord may be pushed so far into the first guide channel that it lies beyond or behind the third opening such that the cord extending from the stiffened end segment is displaced right-angled from the first end segment through the second guide channel, thereby guaranteeing a safe seat and attachment of the cord to the handle portion.

Furthermore, the stiffened end segment is preferably longer than the distance from the first opening to the second opening, such that it has to be pushed out of the first opening after it has been inserted into the second guide channel from the second opening and such that it cannot be pushed directly from the second guide channel into the first guide channel towards its front end. This has the advantage that for one, the stiffened end segment can easily be pushed out of the first opening so that it may be grabbed by user for the further mounting process. And for two, a misassembly of the ear plug system is safely prohibited.

The corded ear plug with the features of present claim 10 is made of the inventive ear plug system described above. The stiffened end segment is arranged within the first guide channel while the flexible cord reaches from the stiffened end segment only through the second guide channel to the outside.

In the following the invention shall be described with reference to the drawings.
- Figure 1: shows an advantageous corded ear plug in a plan view,
- Figure 2: the ear plug in a longitudinal sectional view, and
- Figures 3A to 3C: steps for attaching a cord to the ear plug.

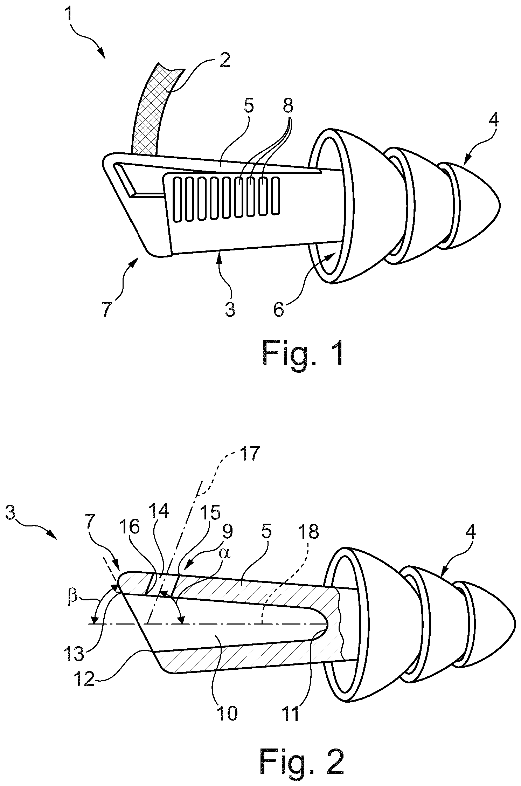

Figure 1 displays in a plan view a corded ear plug 1 that comprises a flexible cord 2 that is attached to an ear plug 3. The ear plug 3 comprises a flexible nose portion 4 which is designed with a number of soundproofing flexible conical lamellaes to be inserted into an ear or ear canal of a human user such that it fits sealing the canal within the ear of the user. The ear plug 3 further comprises a handle portion 5 that extends longitudinally from a first end 6 to a second end 7 and which is attached to the nose portion 4 on the first end 6. The handle portion 3 is designed less flexible than the nose portion 4 such that a user may push or orient the nose portion 4 easier and safely into or in the ear of the user. According to the present example, the handle portion 5 as well as the nose portion 4 are made out of a plastic material, in particular of a rubber material. Advantageously, the nose portion 4 is firmly bonded to the handle portion by use of a material closure or an adhesive bond. The material of the nose portion 4 may extend over a certain length of the handle portion 5 and may comprise, as shown in figure 1, a plurality of protrusions 8 that are designed as rips which are arranged parallel to one another on opposing sides of the handle portion 5 and which are used to provide an advantageous grip for the user of the handle portion 5 for the easy and safe operation of the ear plug 3.

Figure 2 shows another plan view of the ear plug 3 in a partially longitudinal sectional view whereby the section is chosen in the area of the handle portion 5.

The handle portion 5 comprises a fixating structure 9 for attaching the cord 2 to the ear plug 3 without auxiliary measurements such as an adhesive or additional clamping or fixating elements. The fixating structure 9 comprises a first guide channel 10 that is oriented parallel or coaxial to the handle portion 5 such that it extends from a bottom or end 11 within the handle portion in longitudinal direction to the first opening 12 arranged in a front surface 13 of the second end 7. The first guide channel 10 is thus designed as a blind hole in the handle portion 5, the blind hole opening to the front surface 13.

The fixating structure 9 further comprises a second guide channel 14 which is arranged within a mantle wall of the handle portion 5. The second guide channel 14 extends from a second opening 15 that is arranged on the outer mantle surface of the handle portion 5 to a third opening 16 arranged in an inner mantle wall surface and that leads into the first guide channel 10. The second guide channel 14 is arranged close to the front surface 13 and oriented such that it is aligned with the first opening 12. For that reason, the second guide channel 14 may be oriented inclined to the longitudinal extend of the handle portion 5 such that it deviates from a perpendicular orientation. For example, the second guide channel 14 comprises a longitudinal centre line 17 that encloses with a longitudinal centre line 18 of the first guide channel 10 an angle α which is smaller than 90° as shown in figure 2. In order to maximize the angle α, the front surface 13 extend in a plane that is inclined towards the longitudinal centre axis 18 of the first guide channel 10 such that it encloses an angle β that deviates from a perpendicular orientation or from 90°. The angles α and β may be of the same value, but they are chosen such that the longitudinal centre line 17 of the second guide channel 14 reaches through the first opening 12. For this, the longitudinal centre line 17 and the front surface 13 are inclined in opposite directions viewed from a plane perpendicular to the longitudinal centre line 18.

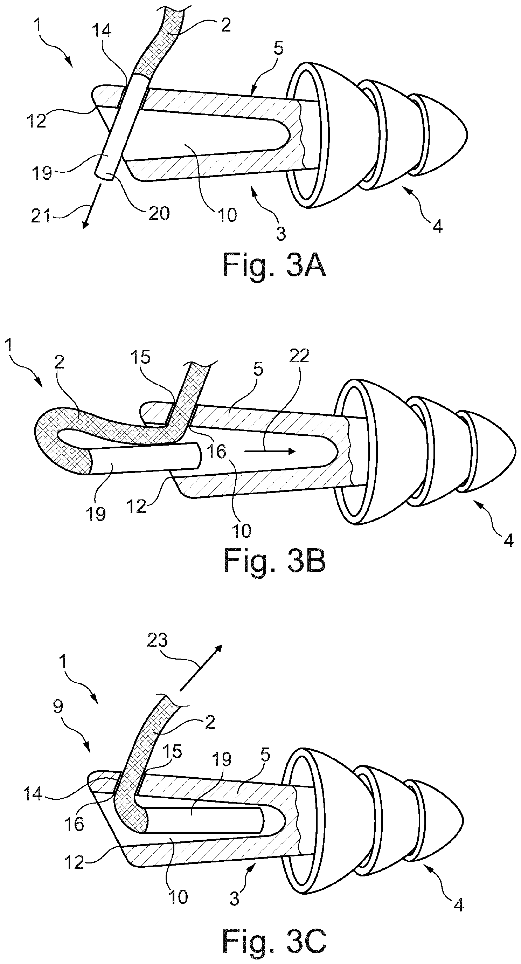

The advantageous fixating structure 9 allows for an easy attachment of the cord 2 to the handle portion 5 of the ear plug 3 in such a way that the cord is mechanically fixed to the handle portion in a safe and reliable manner without auxiliary elements. This shall be described in more detail with reference to figures 3A, B and C.

Figures 3A, B and C display the longitudinal sectional view of figure 2 including the cord 2 in different assembling stages. The cord 2 comprises at least one stiffened end segment 19. In particular, both ends of the cord 2 (the second end is not shown in the figures) are designed equal, such that both ends comprise a stiffened end segment 19 that can be attached to an ear plug 3, respectively.

The stiffened end segments 19 may be formed as aglets 20 made of copper, plastic or bras. The respective aglet 20 is designed as cylindrical mantle wall that surrounds an end of the flexible cord 2 such that the end segment surrounded by the aglet 20 becomes stiff or hard and allows an easy handling of the free end of the cord 2 on one hand and keeps the twine or the cord from unraveling on the other hand. In particular, the cord 2 is designed like a common shoelace.

The stiffened end segment 19 is designed longer than the distance from the first opening 12 to the second opening 15 such that a stiffened end 19 can only be pushed through the second opening 15 through the second guide channel, traversing through the first guide channel 10 and exiting through the first opening 12 as shown in figure 3A. Due to the length of the stiffened end segment 19, the stiffened end segment 19 can be pushed through the second guide channel 14 and the first opening such that the free end of the stiffened end section 19 can be grabbed by a user in order to pull the cord 2 partially through the second and first openings 14, 12 such that the stiffened end section 19 lies fully outside of the handle portion 5 while the flexible cord 2 reaches through the second guide channel and the first opening 12. The cross-section of the second guide channel 14 is thus at least as large as the largest cross-section of the cord 2 in the area of the stiffened end segment 19.

Once, the stiffened end section 19 is located outside of the handle portion 5, the second end segment 19 is pushed back through the first opening into the first guide channel 10 pass the flexile cord 2 as shown in figure 3B and indicated by an arrow 22. The free end segment 19 is pushed into the first guide channel 10 with its free end in front.

The cross-section of the first guide channel 10 is advantageously greater than the cross-section of the stiffened end segment 19 of the cord 2, in particular it is at least as large as the stiffened end section 19 and a flexible part of the cord 2 lying next to each other such that the stiffened end section 19 may be pushed past the flexible segment of the cord 2 that reaches through the second guide channel into the first guide channel 10 as well.

The length of the first guide channel 10 from the third opening 16 until the bottom or end 11 of the first guide channel 10 is designed longer than the longitudinal extend of the stiffened end 19 such that the stiffened end segment 19 may be pushed completely into the first guide channel 10 in particular such that it lies beyond the third opening 16 or between the third opening 16 and the front end 11 within the guide channel 10 as shown in figure 3C. Subsequently, the cord 2 is pulled out of the second guide channel 14 in a direction from the third opening 16 to the second opening 15 as indicated by an arrow 23 such that the remaining part of the cord 2 that was used to arrange the stiffened end segment 19 outside of the handle portion 5 is now pulled out of the second opening 15 until in its longitudinal extend the cord 2 is displaced almost right angled to the stiffened end section 19 as shown in figure 3C.

In this final state of the assembling process, the stiffened end segment 19 is securely arranged within the first guide channel 10. It is not possible for the ear plug 3 to become lose from the cord 2 by itself and the design allows for high tension forces that work on the ear plug 3 and/or the cord 2 without loosening the attachment of the cord 2 on the ear plug 3. The advantageous fixating structure 9, thus, allows for an easy assembling of the corded ear plug 3 and provides a safe and reliable attachment of the cord 2 to the ear plug 3. It is preferably provided that in both ends of the cord 2 a respective ear plug 3 is attached with the help of the above described fixating structure 9.

The presented ear plug 3 together with the cord 2 that comprises different end segments 19 forms an advantageous ear plug system that comprises at least one cord 2, preferably a large number of cords 2 as well as at least two ear plugs 3, preferably a large number of different or equally designed ear plugs 3, so that a user of the ear plug system may choose a certain combination of cord 2 and ear plug 3. By providing a large number of ear plugs 3 and cords 2, in particular cords that have different lengths and ear plugs that have different sizes, in particular with respect to the circumferential design of the nose portion 4, it is possible to choose a fitting combination individually for each person such that the cord 2 has the right length and the nose portion is safely secured soundproofing within the ear of an user. The present invention is set out in the appended claims. The embodiments, examples or aspects according to the present description which do not fall within the scope of said claims are provided for illustrative purposes only and do not form part of the present invention.

## Claims

1. An ear plug (3) for human ears, comprising
- a flexible nose portion (4) for insertion into an ear of a user,
- a handle portion (5) extending longitudinal from a first end (6) to a second end (7), being connected to the nose portion (4) on the first end (6) and comprising a fixation structure (9) for fixating a cord (2) having at least one stiffened end segment (19) to the ear plug (3) on the second end (7),
- the fixation structure (9) comprises a first guide channel (10) oriented at least essentially parallel to the longitudinal extension of the handle portion (5), the first guide channel (10) leading to a first opening (12) at a front surface (13) of the second end (7),
**characterized in that**
- the fixation structure (9) further comprises a second guide channel (14) that extends from a second opening (15) in an outer mantle surface of the handle portion (5) through a third opening (16) into the first guide channel (10) and that is oriented transvers to the first guide channel (10) such that the second guide channel (14) is aligned with the first opening (12), such that the cord (2) can be attached to the fixation structure (9) of the ear plug (2) by pushing the stiffened end segment (19) through the second guide channel (14) from the second opening (15) through the first guide channel (10) and out of the first opening (12) and subsequently pushing it back directly through the first opening (12) into the first guide channel (10) until the stiffened end segment (19) is arranged beyond the third opening (16).

2. The ear plug according to claim 1, **characterized in that** the distance from the second opening (15) to the first opening (12) is shorter than the distance from the first opening (12) to the front end (11) of the first guide channel (10) being opposite to the first opening (12).

3. The ear plug according to one of the preceding claims, **characterized in that** the front surface (13) of the second end (7) extends in a plane that is oriented inclined to a perpendicular orientation to the longitudinal extension of the first guide channel (10) and/or the handle portion (5).

4. The ear plug according to one of the preceding claims, **characterized in that** a cross-section of the first guide channel (10) is larger than a cross-section of the second guide channel (14).

5. The ear plug according to one of the preceding claims, **characterized in that** the front surface (13) of the second end (7) extends in a plane that is inclined such that the second guide channel (14) is at least essentially aligned with the first opening (12).

6. The ear plug according to one of the preceding claims, **characterized in that** the first guide channel (10) is conical or cylindrical in shape.

7. An ear plug system for human beings with at least one cord (2) having at least one stiffened end segment (19) and with at least one ear plug (3) according to one of claims 1 to 6.

8. The ear plug system according to claims 2 and 7, **characterized in that** the stiffened end segment (19) and/or the the first guide channel (10) are designed such that the stiffened end segment (19) can fully be arranged between the third opening (16) and the front end (11).

9. The ear plug system according to claim 7 or 8, **characterized in that** the stiffened end segment (19) is longer than the distance from the first opening (12) to the second opening (15).

10. A corded ear plug made of the ear plug system according to one of claims 7 to 9, whereby the stiffened end segment (19) of the cord (2) is arranged within the first guide channel (10) while the cord (2) reaches through the second guide channel (14) into the first guide channel (10) with a flexible cord segment.

## Patentansprüche

1. Ein Ohrstöpsel (3) für menschliche Ohren, umfassend
- einen flexiblen Frontabschnitt (4) zum Einführen in ein Ohr eines Benutzers,
- einen Griffabschnitt (5), der sich in Längsrichtung von einem ersten Ende (6) zu einem zweiten Ende (7) erstreckt, mit dem Frontabschnitt (4) an dem ersten Ende (6) verbunden ist und eine Befestigungsstruktur (9) zum Befestigen einer Schnur (2) mit zumindest einem versteiften Endsegment (19) an dem Ohrstöpsel (3) an dem zweiten Ende (7) umfasst,
- wobei die Befestigungsstruktur (9) einen ersten Führungskanal (10) umfasst, der zumindest im Wesentlichen parallel zu der Längserstreckung des Griffabschnitts (5) ausgerichtet ist, wobei der erste Führungskanal (10) zu einer ersten Öffnung (12) in einer Stirnfläche (13) des zweiten Endes (7) führt,
**dadurch gekennzeichnet, dass**
- die Befestigungsstruktur (9) ferner einen zweiten Führungskanal (14) umfasst, der sich von einer zweiten Öffnung (15) in einer äußeren Mantelfläche des Griffabschnitts (5) durch eine dritte Öffnung (16) in den ersten Führungskanal (10) erstreckt und der quer zu dem ersten Führungskanal (10) ausgerichtet ist, so dass der zweite Führungskanal (14) mit der ersten Öffnung (12) fluchtend ausgerichtet ist, so dass die Schnur (2) an der Befestigungsstruktur (9) des Ohrstöpsels (2) befestigbar ist, indem das versteifte Endsegment (19) durch den zweiten Führungskanal (14) von der zweiten Öffnung (15) durch den ersten Führungskanal (10) und aus der ersten Öffnung (12) herausgeschoben und anschließend direkt durch die erste Öffnung (12) in den ersten Führungskanal (10) zurückgeschoben wird, bis das versteifte Endsegment (19) jenseits der dritten Öffnung (16) angeordnet ist.

2. Ohrstöpsel nach Anspruch 1, **dadurch gekennzeichnet, dass** der Abstand von der zweiten Öffnung (15) zu der ersten Öffnung (12) kürzer ist als der Abstand von der ersten Öffnung (12) zu dem der ersten Öffnung (12) gegenüberliegenden vorderen Ende (11) des ersten Führungskanals (10).

3. Ohrstöpsel nach einem der vorhergehenden Ansprüche, **dadurch gekennzeichnet, dass** die Stirnfläche (13) des zweiten Endes (7) in einer Ebene verläuft, die schräg zu einer senkrechten Ausrichtung zu der Längserstreckung des ersten Führungskanals (10) und/oder des Griffabschnitts (5) ausgerichtet ist.

4. Ohrstöpsel nach einem der vorhergehenden Ansprüche, **dadurch gekennzeichnet, dass** ein Querschnitt des ersten Führungskanals (10) größer ist als ein Querschnitt des zweiten Führungskanals (14).

5. Ohrstöpsel nach einem der vorhergehenden Ansprüche, **dadurch gekennzeichnet, dass** die Stirnfläche (13) des zweiten Endes (7) in einer Ebene verläuft, die derart schräg ist, dass der zweite Führungskanal (14) zumindest im Wesentlichen mit der ersten Öffnung (12) fluchtend ausgerichtet ist.

6. Ohrstöpsel nach einem der vorhergehenden Ansprüche, **dadurch gekennzeichnet, dass** der erste Führungskanal (10) eine konische oder zylindrische Form aufweist.

7. Ohrstöpselsystem für Menschen mit zumindest einer Schnur (2) mit zumindest einem versteiften Endsegment (19) und mit zumindest einem Ohrstöpsel (3) nach einem der Ansprüche 1 bis 6.

8. Ohrstöpselsystem nach den Ansprüchen 2 und 7, **dadurch gekennzeichnet, dass** das versteifte Endsegment (19) und/oder der erste Führungskanal (10) derart ausgebildet sind, dass das versteifte Endsegment (19) vollständig zwischen der dritten Öffnung (16) und dem vorderen Ende (11) anordenbar ist.

9. Ohrstöpselsystem nach Anspruch 7 oder 8, **dadurch gekennzeichnet, dass** das versteifte Endsegment (19) länger ist als der Abstand von der ersten Öffnung (12) zu der zweiten Öffnung (15).

10. Ohrstöpsel mit Schnur hergestellt aus dem Ohrstöpselsystem nach einem der Ansprüche 7 bis 9, wobei das versteifte Endsegment (19) der Schnur (2) innerhalb des ersten Führungskanals (10) angeordnet ist, während die Schnur (2) mit einem flexiblen Schnursegment durch den zweiten Führungskanal (14) in den ersten Führungskanal (10) reicht.

## Revendications

1. Un bouchon d'oreille (3) pour les oreilles humaines, comprenant
- une partie frontale flexible (4) destinée à être insérée dans l'oreille d'un utilisateur,
- une partie poignée (5) s'étendant longitudinalement d'une première extrémité (6) à une seconde extrémité (7), étant reliée à la partie frontale (4) sur la première extrémité (6) et comprenant une structure de fixation (9) pour fixer un cordon (2) ayant au moins un segment d'extrémité rigide (19) au bouchon d'oreille (3) sur la seconde extrémité (7),
- la structure de fixation (9) comprend un premier canal de guidage (10) orienté au moins essentiellement parallèlement à l'extension longitudinale de la partie poignée (5), le premier canal de guidage (10) menant à une première ouverture (12) sur une surface frontale (13) de la deuxième extrémité (7),
**caractérisé en ce que**
- la structure de fixation (9) comprend en outre un deuxième canal de guidage (14) qui s'étend d'une deuxième ouverture (15) dans une surface extérieure du revêtement de la partie poignée (5) à travers une troisième ouverture (16) dans le premier canal de guidage (10) et qui est orienté transversalement au premier canal de guidage (10) de telle sorte que le deuxième canal de guidage (14) est aligné avec la première ouverture (12), de sorte que le cordon (2) puisse être attaché à la structure de fixation (9) du bouchon d'oreille (2) en poussant le segment d'extrémité rigide (19) à travers le deuxième canal de guidage (14) depuis la deuxième ouverture (15) à travers le premier canal de guidage (10) et hors de la première ouverture (12) et en le repoussant ensuite directement à travers la première ouverture (12) dans le premier canal de guidage (10) jusqu'à ce que le segment d'extrémité rigide (19) soit disposé au-delà de la troisième ouverture (16).

2. Bouchon d'oreille selon la revendication 1, **caractérisé en ce que** la distance entre la deuxième ouverture (15) et la première ouverture (12) est plus courte que la distance entre la première ouverture (12) et l'extrémité frontale (11) du premier canal de guidage (10) se trouvant à l'opposé de la première ouverture (12).

3. Bouchon d'oreille selon l'une des revendications précédentes, **caractérisé en ce que** la surface frontale (13) de la deuxième extrémité (7) s'étend dans un plan orienté de manière inclinée par rapport à une orientation perpendiculaire à l'extension longitudinale du premier canal de guidage (10) et/ou de la partie poignée (5).

4. Bouchon d'oreille selon l'une des revendications précédentes, **caractérisé en ce qu'**une section transversale du premier canal de guidage (10) est plus grande qu'une section transversale du second canal de guidage (14).

5. Bouchon d'oreille selon l'une des revendications précédentes, **caractérisé en ce que** la surface frontale (13) de la seconde extrémité (7) s'étend dans un plan incliné de sorte que le second canal de guidage (14) est au moins essentiellement aligné avec la première ouverture (12).

6. Bouchon d'oreille selon l'une des revendications précédentes, **caractérisé en ce que** le premier canal de guidage (10) est de forme conique ou cylindrique.

7. Système de bouchon d'oreille pour êtres humains avec au moins un cordon (2) ayant au moins un segment d'extrémité rigide (19) et avec au moins un bouchon d'oreille (3) selon l'une des revendications 1 à 6.

8. Système de bouchons d'oreille selon les revendications 2 et 7, **caractérisé en ce que** le segment d'extrémité rigide (19) et/ou le premier canal de guidage (10) sont conçus de manière à ce que le segment d'extrémité rigide (19) puisse être entièrement disposé entre la troisième ouverture (16) et l'extrémité frontale (11).

9. Système de bouchons d'oreille selon la revendication 7 ou 8, **caractérisé en ce que** le segment d'extrémité rigide (19) est plus long que la distance entre la première ouverture (12) et la deuxième ouverture (15).

10. Bouchon d'oreille à cordon constitué du système de bouchon d'oreille selon l'une des revendications 7 à 9, le segment d'extrémité rigide (19) du cordon (2) étant disposé à l'intérieur du premier canal de guidage (10) tandis que le cordon (2) parvient à travers le deuxième canal de guidage (14) dans le premier canal de guidage (10) avec un segment de cordon flexible.
